(19) Europäisches Patentamt

European Patent Office

Office européen des brevets

(11) **EP 1 516 084 B1**

(12) **EUROPÄISCHE PATENTSCHRIFT**

(45) Veröffentlichungstag und Bekanntmachung des Hinweises auf die Patenterteilung:
**27.09.2006 Patentblatt 2006/39**

(21) Anmeldenummer: **03735564.1**

(22) Anmeldetag: **06.06.2003**

(51) Int Cl.:
*D06M 15/263* (2006.01)    *A61L 15/60* (2006.01)
*C08J 3/00* (2006.01)

(86) Internationale Anmeldenummer:
**PCT/EP2003/005939**

(87) Internationale Veröffentlichungsnummer:
**WO 2003/104543 (18.12.2003 Gazette 2003/51)**

(54) **VERFAHREN ZUM BINDEN VON TEILCHENFORMIGEN, WASSERABSORBIERENDEN, SAUREGRUPPEN ENTHALTENDEN POLYMEREN AN EIN TRAGERMATERIAL**

METHOD FOR BINDING PARTICULATE, WATER-ABSORBING, ACID GROUP-CONTAINING POLYMERS TO A BASE MATERIAL

PROCEDE DE FIXATION DE POLYMERES PARTICULAIRES HYDROABSORBANTS COMPORTANT DES GROUPES ACIDES SUR UN MATERIAU SUPPORT

(84) Benannte Vertragsstaaten:
**AT BE BG CH CY CZ DE DK EE ES FI FR GB GR HU IE IT LI LU MC NL PT RO SE SI SK TR**

(30) Priorität: **11.06.2002   DE 10225944**

(43) Veröffentlichungstag der Anmeldung:
**23.03.2005   Patentblatt 2005/12**

(73) Patentinhaber: **BASF Aktiengesellschaft**
**67056 Ludwigshafen (DE)**

(72) Erfinder:
• **FUNK, Rüdiger**
  **65527 Niedernhausen (DE)**
• **HOSS, Ulrike**
  **65830 Kriftel (DE)**

(56) Entgegenhaltungen:
**EP-A- 0 205 674        EP-A- 0 442 185**
**WO-A-01/56625          WO-A-94/04351**
**WO-A-94/04352**

EP 1 516 084 B1

**Beschreibung**

Die Erfindung betrifft ein Verfahren zum Binden von teilchenförmigen, wasserabsorbierenden, Säuregruppen enthaltenden, vernetzten Polymeren an ein Trägermaterial mit Hilfe von Aminogruppen aufweisenden Verbindungen.

Teilchenförmige, wasserabsorbierende, Säuregruppen enthaltende, vernetzte Polymere, die ein Vielfaches ihres Gewichts an Wasser absorbieren können, sind unter der Bezeichnung Superabsorber bekannt bzw. werden auch superabsorbierende Polymere (SAP) genannt. Sie werden beispielsweise in Hygieneartikeln wie Windeln, Tampons oder Verbänden, zur Absorption von Körperflüssigkeiten eingesetzt. Die teilchenförmigen Superabsorber bestehen z.B. aus vernetzten Polyacrylsäuren, die zu mindestens 50 Mol-% neutralisiert sind. Die Oberfläche der Teilchen ist meistens nachvernetzt worden. Bei der Handhabung bzw. dem Gebrauch von Hygieneartikeln können sich die darin enthaltenen Superabsorber von den Cellulosefasern separieren, so daß sie nicht mehr gleichmäßig im Hygieneartikel verteilt sind. Dadurch wird aber die Aufnahmekapazität und Aufnahmegeschwindigkeit von Körperflüssigkeiten nachteilig beeinflußt. Um in einem Hygieneartikel teilchenförmige Superabsorber an die Cellulosefasern zu binden und sie auf diese Weise zu fixieren, hat man bereits Polyaluminiumchlorid als Binder verwendet, vgl. EP-A-0 442 185.

Aus der US-A-5 589 256 ist bekannt, teilchenförmige Superabsorber mit Hilfe von polymeren und nicht-polymeren Bindern an Fasern, vorzugsweise Cellulosefasern, zu fixieren und die Mischung zu Vliesen zu verarbeiten. Geeignete Binder sind beispielsweise Polypropylenglykol, Polyacrylsäure, Polyglycin, Polyethylenimin, Polyvinylpyridin, Butandiol, Ethanolamin, Propylenglykol und Ascorbinsäure. Vliese, bei denen ein Superabsorber mit Hilfe eines Binders an Cellulosefasern gebunden ist, können gegebenenfalls durch Einwirkung von Drücken, z.B. in einem Kalander, verdichtet werden. Man erhält dadurch relativ dünne absorbierende Artikel, die beispielsweise in Hygieneartikeln eingesetzt werden.

Aus der US-A-6 099 950 sind absorbierende Polymere mit verbesserten Absorptionseigenschaften bekannt. Die Polymeren sind oberflächennachvernetzt und mit einem kationischen Polymer, das ein mittleres Molekulargewicht von mindestens 500 hat, überzogen. Geeignete kationische Polymere sind beispielsweise Polyvinylamine, Polyallylamine, Polyethylenimine und modifizierte Polyethylenimine. Wie aus den Beispielen ersichtlich ist, wird Polyallylamin als kationisches Polymer eingesetzt. Die mit Polyallylamin behandelten Superabsorber haben gegenüber den nicht behandelten Produkten deutlich erhöhte SFC-Werte (Salt-Flow-Conductivity) und BBS-Werte (Ball Burst Strength) und einen niedrigeren Gehalt an extrahierbaren Anteilen.

Aus der WO-A-99/44648 sind offenzellige Schaumstoffe aus vernetzten Polyacrylaten bekannt, deren Säuregruppen nach der Polymerisation zu mindestens 20 Mol-% mit einem Alkanolamin neutralisiert worden sind. Solche Schaumstoffe sind weich und flexibel. Ihre Oberfläche ist jedoch klebrig. Um die Klebrigkeit zu vermindern bzw. aufzuheben, beschichtet man sie meistens mit feinteiligen Pulvern wie Siliciumdioxid, Talkum oder Silikaten.

Der vorliegenden Erfindung liegt die Aufgabe zugrunde, ein weiteres Verfahren zum Binden von teilchenförmigen, wasserabsorbierenden, Säuregruppen enthaltenden Polymeren an ein Trägermaterial zur Verfügung zu stellen, wobei man gegenüber den bekannten Verfahren eine möglichst festere Bindung der Polymerteilchen an das Trägermaterial erhält.

Die Aufgabe wird erfindungsgemäß gelöst mit einem Verfahren zum Binden von teilchenförmigen, wasserabsorbierenden, Säuregruppen enthaltenden Polymeren an ein Trägermaterial mit Hilfe von Aminogruppen aufweisenden Verbindungen, wenn man das Trägermaterial mit teilchenförmigen, wasserabsorbierenden, Säuregruppen enthaltenden Polymeren in Kontakt bringt, deren Säuregruppen zu 0 bis höchstens 55 Mol-% mit Alkalimetall- und/oder Ammoniumbasen neutralisiert sind, und dann den Neutralisationsgrad dieser Polymeren durch Behandlung mit Aminogruppen enthaltenden Verbindungen auf mindestens 60 Mol-% erhöht.

Gegenstand der Erfindung ist außerdem die Verwendung von Aminogruppen aufweisenden Verbindungen aus der Gruppe der Alkanolamine, Ethylenimin- und/oder Vinylamineinheiten enthaltenden Verbindungen zur Neutralisation und zum Binden von teilchenförmigen, wasserabsorbierenden, Säuregruppen enthaltenden Polymeren, die zu 0 bis höchstens 55 Mol-% mit Alkalimetall- und/oder Ammoniumbasen neutralisiert sind, an ein Trägermaterial.

Meistens geht man von solchen teilchenförmigen, wasserabsorbierenden, Säuregruppen enthaltenden, vernetzten Polymeren (teilchenförmige SAP) aus, deren Säuregruppen zu weniger als 50 Mol-% neutralisiert sind. Die teilchenförmigen SAP, die zunächst mit einem Trägermaterial in Kontakt gebracht werden, haben vorzugsweise einen Neutralisationsgrad von 0 bis 45 Mol-%. Besonders bevorzugt ist ein Bereich für den Neutralisationsgrad der teilchenförmigen SAP von 15 bis 40 Mol-%. Außerdem können mit Vorteil nicht neutralisierte teilchenförmige SAP eingesetzt werden, bei denen sämtliche Säuregruppen in Form von freien Carboxylgruppen vorliegen.

Teilchenförmige, wasserabsorbierende, Säuregruppen enthaltende, vernetzte Polymere, deren Säuregruppen zu 0 bis höchstens 55 Mol-% mit Alkalimetall- und/oder Ammoniumbasen neutralisiert sind, sind bekannt, vgl. EP-A-0 205 674, EP-A-0 530 438 und US-A-5 145 906. Solche teilchenförmigen SAP sind beispielsweise dadurch erhältlich, daß man die Säuregruppen enthaltenden Monomeren in nicht neutralisierter oder in partiell neutralisierter Form in Gegenwart eines Vernetzers und eines Polymerisationsinitiators in wäßrigem Milieu polymerisiert und das erhaltene Hydrogel zerkleinert. Falls die bei der Polymerisation eingesetzten Säuregruppen tragenden Monomeren noch nicht den Neutralisationsgrad aufweisen, den die erfindungsgemäß zu verwendenden SAP haben sollen, kann man die bei der Polymerisation anfallenden Gele durch Reaktion mit Alkalimetallbasen und/oder Ammoniumbasen neutralisieren. Als Alkalimetallbasen werden vorzugsweise Natronlauge oder Kalilauge verwendet. Geeignete Ammoniumbasen sind beispielsweise Ammoniak, primäre, sekundäre und/oder tertiäre Amine.

Um Hydrogele mit verbesserten Absorptionseigenschaften und verbesserter mechanischer Stabilität herzustellen, werden bekanntlich teilchenförmige SAP einer Oberflächennachvernetzung unterworfen, vgl. beispielsweise US-A-5 145 906 und US-A-5 945 495. Geeignete Säuregruppen enthaltende Monomere zur Herstellung von SAP werden beispielsweise in der US-A-5 945 495, Spalte 5 genannt. Ein besonders bevorzugtes Monomer dieser Art ist Acrylsäure. Außer den durch Polymerisieren der den SAP zugrundeliegenden Monomeren erhältlichen Polymerisaten kommen auch Pfropfpolymerisate von Säuregruppen enthaltenden Monomeren auf beispielsweise Polysacchariden wie Stärke oder auf Polyalkylenglykolen oder hydrophilen Polyestern in Betracht, vgl. US-A-5 945 495, Spalten 5 und 6. Bevorzugt eingesetzte teilchenförmige SAP sind oberflächennachvernetze, teilchenförmige, vernetzte Polyacrylsäuren mit einem Neutralisationsgrad von 0 bis höchstens 55 Mol-%, vorzugsweise bis weniger als 50 Mol-%. Der pH-Wert der teilchenförmigen SAP beträgt beispielsweise 3 bis 6 und liegt vorzugsweise in dem Bereich von 4 bis 5,7 und beträgt besonders bevorzugt 4,5 bis 5,5.

Der mittlere Teilchendurchmesser der teilchenförmigen SAP beträgt beispielsweise 50 bis 2000 μm, vorzugsweise 100 bis 850 μm und insbesondere 150 bis 700 μm.

Die oben beschriebenen teilchenförmigen SAP mit einem Neutralisationsgrad von höchstens 55 Mol-% werden an ein Trägermaterial gebunden. Als Trägermaterial ist jede Matrix geeignet, die die hochquellfähigen teilchenförmigen SAP aufnehmen kann. Ein Beispiel hierfür ist eine Matrix aus Fasern, z.B. aus natürlichen und/oder synthetischen Fasern. Die Fasern können beispielsweise mit den teilchenförmigen SAP gemischt und in Form der Mischung zu einem Vlies verarbeitet werden. Die teilchenförmigen SAP können jedoch auch in ein Vlies eingearbeitet werden, das beispielsweise aus Cellulosefasern oder einer Mischung aus Cellulosefasern und synthetischen Fasern besteht. Ebenso ist es möglich, die teilchenförmigen SAP mit Fasern oder Vliesen aus jeweils synthetischen Polymeren als Trägermaterial zu einer Matrix zu verarbeiten. Weitere geeignete Trägermaterialien sind offenporige Schäume aus beispielsweise Melamin-Formaldehyd-Kondensaten, aliphatischen Polyurethanen oder vernetzten Polyacrylaten. Außerdem kommen Folien aus verschiedenen Materialien wie Polyethylen, Polypropylen, Polyamiden oder Polyurethanen als Trägermaterialien in Betracht. Bevorzugt werden Cellulosefasern und Vliese aus Cellulosefasern als Trägermaterial verwendet. Außer Vliesen eignen sich auch Gewebe oder Tissues als Trägermaterial, das wasserdurchlässig oder auch wasserundurchlässig sein kann.

Als Trägermaterial kommen auch Verbundmaterialien in Betracht, die beispielsweise durch Verbinden von mindestens zwei Folien, Geweben oder Vliesen entstehen. Die Verbunde können dabei so ausgebildet sein, daß sie eine Vielzahl an Kammern aufweisen, die die teilchenförmigen SAP aufnehmen können. Ein zweischichtiger Verbund kann beispielsweise aus einer wasserundurchlässigen Folie aus Polyethylen oder Polypropylen und einer Schicht aus einem Vlies aus Cellulosefasern bestehen und die teilchenförmigen SAP zwischen beiden Schichten enthalten. Ebenso sind Verbunde möglich, deren Ober- und Unterseite aus z.B. Cellulosefasern bestehen und die teilchenförmige SAP in gleichmäßiger oder auch in heterogener Verteilung zwischen beiden Lagen oder in beiden Vliesen enthalten. Verfahren zur Herstellung solcher Verbundmaterialien sind bekannt. Die Herstellung kann entweder diskontinuierlich oder kontinuierlich erfolgen.

Der Anteil der teilchenförmigen SAP in der Kombination aus Trägermaterial und SAP beträgt beispielsweise 30 bis 95, vorzugsweise 50 bis 95 Gew.-% und liegt besonders bevorzugt bei 60 bis 95 und meistens bei 80 bis 95 Gew.-%. In Kombinationen mit besonders hoher Speicherwirkung für wäßrige Flüssigkeiten beträgt die Menge an teilchenförmigen SAP beispielsweise 90 bis 95 Gew.-%.

Die teilchenförmigen SAP werden durch Behandlung mit Aminogruppen enthaltenden Verbindungen, die basisch reagieren, unter Erhöhung des Neutralisationsgrades der erfindungsgemäß einzusetzenden teilchenförmigen SAP auf mindestens 60 Mol-% an ein Trägermaterial gebunden. Als Aminogruppen enthaltende Verbindungen kommen beispielsweise Alkanolamine in Betracht.

Die verwendeten Alkanolamine können in ihrer Struktur primär, sekundär, tertiär oder quaternär sein und einwertige, mehrwertige oder polyfunktionelle Basen darstellen. Die Alkanolamine können zusätzlich zu ihren Amino- und Hydroxylgruppen weitere funktionelle Gruppen wie z.B. Ester-, Urethan-, Ether-, Thioether-, Harnstoffgruppe usw. tragen. Eingesetzt werden können z.B. niedermolekulare Verbindungen wie Triethanolamin, Methyldiethanolamin, Dimethylethanolamin, Ethanolamin, N-Hydroxyethylmorpholin, Dimethylaminodiglycol, N,N,N',N'-Tetra-(hydroxyethyl)-ethylendiamin, N,N,N',N'-Tetra-(hydroxypropyl)-ethylendiamin, Dimethylaminotriglycol, Diethylaminoethanol, 3-Dimethylamino-1,2-propandiol, Triisopropanolamin, Diisopropylaminoethanol, Cholinhydroxid, Cholincarbonat, 2-tert.-Butylaminoethanol, Tris(oxymethyl)aminomethan, 3-Amino-1-propanol, Isopropanolamin, 2-(2-Aminoethoxy)ethanol, 2-Amino-2-methyl-1-propanol oder aber Oligomere oder Polymere, die durch Umsetzung von Aminogruppen tragenden Polymerisaten oder Kondensaten wie z.B. Polyethyleniminen oder Polyvinylaminen mit Ethylenoxid, Propylenoxid, Glycidol oder anderen Epoxiden erhältlich sind.

Vorzugsweise kommen Triethanolamin, Methyldiethanolamin, Dimethylaminodiglycol, Dimethylethanolamin, Ethanolamin, N,N,N',N'-Tetra-(hydroxyethyl)-ethylendiamin oder deren Mischungen in Betracht. Besonders bevorzugt wird Triethanolamin eingesetzt.

Als Aminogruppen enthaltende Verbindungen, die basisch reagieren, können auch Polymere verwendet werden, z.B. Ethylenimineinheiten und/oder Vinylamineinheiten enthaltende Polymere. Ethylenimineinheiten enthaltende Polymere werden beispielsweise durch Polymerisieren von Ethylenimin in wäßrigem Medium in Gegenwart saurer Katalysatoren oder durch Pfropfen von beispielsweise Polyamidoaminen mit Ethylenimin hergestellt. Vinylamineinheiten enthaltende Polymere werden durch Hydrolyse von Polymeren hergestellt, die ein offenkettiges N-Vinylcarbonsäureamid wie N-Vinylformamid oder N-Vinylacetamid einpolymerisiert enthalten, vgl. US-A-4,421,602, US-A-5,334,287, EP-A-0 216 387, US-A-5,981,689, WO-A-00/63295 und US-A-6,121,409. Vorzugsweise geht man von Homopolymerisaten des N-Vinylformamids aus und hydrolysiert die polymeren N-Vinylformamide unter Abspaltung von z.B. 5 bis 100 Mol-% Formylgruppen zu Vinylamineinheiten enthaltenden Polymerisaten.

Die Molmassen Mw der obengenannten Polymeren betragen mindestens 300, meistens mindestens 500. Die Molmassen Mw der Polyethylenimine und der Vinylamineinheiten enthaltenden Polymeren liegt vorzugsweise in dem Bereich von 1000 bis 2 Millionen, und insbesondere bei 1500 bis 500 000.

Um teilchenförmige SAP an ein Trägermaterial zu binden, bringt man zunächst das Trägermaterial mit teilchenförmigen, wasserabsorbierenden, Säuregruppen enthaltenden Polymeren in Kontakt, deren Säuregruppen zu 0 bis höchstens 55 Mol-% mit Alkalimetall- und/oder Ammoniumbasen neutralisiert sind, und erhöht dann den Neutralisationsgrad dieser Polymeren durch Behandlung mit Aminogruppen enthaltenden Verbindungen auf mindestens 60 Mol-% erhöht. Beispielsweise kann man auf ein Vlies aus natürlichen und/oder synthetischen Fasern zunächst teilchenförmigen SAP mit einem Neutralisationsgrad von 0 bis 55 Mol-% aufbringen und den Neutralisationsgrad der teilchenförmigen SAP dann durch Besprühen mit einer Lösung einer Aminogruppen enthaltenden Verbindung auf mindestens 60 Mol-% erhöhen. Dadurch wird der teilchenförmige SAP klebrig und haftet an den Fasern, mit denen er in Kontakt ist. Nach dem erfindungsgemäßen Verfahren können alle bekannten Hygieneartikel hergestellt werden, wenn man den im Hygieneartikel enthaltenen absorbierenden Kern, sofern er teilchenförmigen SAP mit einem Neutralisationsgrad von höchstens 55 Mol-% enthält, mit einer basischen, Aminogruppen enthaltenen Verbindung in der Weise behandelt, daß der Neutralisationsgrad der teilchenförmigen SAP auf mindestens 60 Mol-% erhöht wird.

Die zur Neutralisation verwendeten Aminogruppen enthaltenden Verbindungen werden vorzugsweise in Form von Lösungen eingesetzt. Geeignete Lösemittel sind beispielsweise Wasser, Alkohole wie Methanol, Ethanol, Isopropanol, n-Propanol und tert.-Butanol, Ketone wie Aceton oder Methylethylketon, Ester wie Ethylacetat oder Ether wie Dioxan oder Tetrahydrofuran. In manchen Fällen ist es vorteilhaft, Mischungen aus zwei oder mehreren Lösemitteln für die Binder einzusetzen, z.B. Mischungen aus Wasser und Ethanol, Mischungen aus Wasser und Isopropanol oder Mischungen aus Wasser und Aceton. Bevorzugt eingesetztes Lösemittel ist Wasser. Die Konzentration der Binder im Lösemittel bzw. im Lösemittelgemisch beträgt beispielsweise 1 bis 100, vorzugsweise 70 bis 95 Gew.-%. Bei flüssigem Binder, wie z.B. Triethanolamin wird der Binder vorzugsweise ohne Lösungsmittel verwendet.

Zusammen mit teilchenförmigen SAP können in Hygieneartikeln beispielsweise Mittel zur Geruchskontrolle eingesetzt werden. Solche Substanzen können nach dem erfindungsgemäßen Verfahren an die teilchenförmigen absorbierenden Polymeren und damit an die Trägermaterialien gebunden werden. Vorteilhaft ist hier die Verwendung von teilchenförmigen Mitteln, die eine Geruchskontrolle beim Gebrauch des Hygieneartikels bewirken, z.B. anorganische Substanzen mit großer Oberfläche wie feinteilige amorphe Polykieselsäuren (Aerosil®), Bentonite, Zeolithe und Aktivkohle. Außerdem können die für eine Geruchsinhibierung bekannten organischen Substanzen wie Cyclodextrine usw. in den üblicherweise eingesetzten Mengen verwendet werden.

Nach der Behandlung der absorbierenden Komposition, die zumindest aus einem Trägermaterial und einem teilchenförmigen SAP mit einem Neutralisationsgrad von 0 bis 55 Mol-% besteht, mit einem Bindemittel, vorzugsweise Triethanolamin, wird sie mit Hilfe konventioneller Techniken getrocknet, z.B. durch Einwirkung von Infrarotbestrahlung, durch Erhitzen mit Mikrowellenbestrahlung oder Erhitzen in einem heißen Gasstrom oder - gegebenenfalls zusätzlich - Einstellen eines verminderten Drucks. Um die Lösemittel aus der absorbierenden Komposition zu entfernen erhitzt man das Material beispielsweise auf Temperaturen in dem Bereich von 50 bis 120°C.

Die absorbierende Komposition kann jedoch auch gegebenenfalls zusätzlich auf noch höhere Temperaturen erhitzt werden, wodurch die Formbeständigkeit der Absorptionsschicht im feuchten Zustand weiter verbessert werden kann. Diese Art der Hitzbehandlung wird beispielsweise mit einem heißen Gasstrom oder durch Bestrahlen mit Infrarotstrahlung bei Temperaturen bis zu 230°C durchgeführt. Die Temperaturen bei der Hitzbehandlung der Kompositmaterialien liegen vorzugsweise bei 100 bis 200°C und insbesondere bei 100 bis 180°C. Die Dauer der Hitzebehandlung hängt von verschiedenen Faktoren ab, z.B. der Art der synthetischen Fasern, der Zusammensetzung des Gemisches aus teilchenförmigem SAP und Trägermaterial und der Geschwindigkeit bei der Herstellung des Hygieneartikels. Sie beträgt beispielsweise 0,5 Sekunden bis 3 Minuten und liegt meistens in dem Bereich von 1 Sekunde bis 1 Minute.

Das erfindungsgemäße Verfahren wird vorzugsweise in den Herstellungsprozeß des Hygieneartikels integriert, wobei man den Neutralisationsgrad der teilchenförmigen SAP erst nach der Einarbeitung der teilchenförmigen SAP in den Verbund oder gleichzeitig mit dem Zusammenbringen von teilchenförmigen SAP und Trägermaterial vornimmt. Bekannte Hygieneartikel haben beispielsweise folgenden Aufbau:

(A) eine obere flüssigkeitsdurchlässige Abdeckung

(B) eine untere flüssigkeitsundurchlässige Schicht

(C) einen zwischen (A) und (B) befindlichen Kern, enthaltend

(i) 10-100 Gew.-% des Hydrogel-formenden Polymers

(ii) 0-90 Gew.-% hydrophiles Fasermaterial

(D) gegebenenfalls eine sich unmittelbar oberhalb und unterhalb des Kerns (C) sich befindende Tissueschicht und

(E) gegebenenfalls eine zwischen (A) und (C) sich befindende Aufnahmeschicht.

Unter Hygieneartikel sind dabei sowohl Inkontinenzeinlagen und Inkontinenzhosen für Erwachsene als auch Windeln für Babys zu verstehen.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugt Materialien sind Polyester, Rayon und deren Blends, Polyethylen und Polypropylen.

Die flüssigkeitsundurchlässige Schicht (B) besteht in der Regel aus einer Folie aus Polyethylen oder Polypropylen.

Der Kern (C) enthält neben dem erfindungsgemäßen Hydrogel-formenden Polymer (i) hydrophiles Fasermaterial (ii). Unter hydrophil ist zu verstehen, daß sich wäßrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1-200 $\mu$m, bevorzugt 10-100 $\mu$m. Darüberhinaus haben die Fasern eine Mindestlänge von 1 mm.

Der Anteil des hydrophilen Fasermaterials bezogen auf die Gesamtmenge des Kerns beträgt bevorzugt 20-80 Gew.-%, besonders bevorzugt 40-70 Gew.-%. Solche Windeln werden beispielsweise in der EP-A-0 316 518 beschrieben.

Aufgrund der starken Fixierung der teilchenförmigen SAP und gegebenenfalls weiterer Bestandteile an das Trägermaterial weisen die nach dem erfindungsgemäßen Verfahren hergestellten absorbierenden Kompositionen eine außerordentliche Stabilität im trockenen und im nassen Zustand auf. Das teilchenförmige absorbierende Material, das im Hygieneartikel erfindungsgemäß fixiert ist, verschiebt sich beim Gebrauch oder dem Transport des Hygieneartikels nicht. Er zeichnet sich durch eine große Naßfestigkeit und Formstabilität aus. In hoher Konzentration eingelagerte absorbierende teilchenförmige Polymere haben ein hohes Absorptionsvermögen, eine hohe Gelfestigkeit, Permeabilität und Retention. Sie weisen darüber hinaus verbesserte Geruchseigenschaften beim Gebrauch der Hygieneartikel auf. Da der pH-Wert der teilchenförmigen SAP nach dem Fixieren im Hygieneartikel unter 7,0 liegt, haben die absorbierenden Polymeren eine antimikrobielle Wirkung, so daß in den meisten Fällen auf den Zusatz von geruchsinhibierenden Substanzen verzichtet werden kann. Beim Gebrauch der Hygieneartikel werden wegen des fast gleichbleibenden pH-Wertes im Superabsorber Hautreizungen und Hautirritationen praktisch vermieden.

Testmethoden

Zentrifugenretentionskapazität (CRC - Centrifuge Retention Capacity)

Bei dieser Methode wird die freie Quellbarkeit des Hydrogels im Teebeutel bestimmt. Zur Bestimmung der CRC werden 0,2000 ± 0,0050 g getrocknetes Hydrogel (Kornfraktion 106-850 $\mu$m) in einem 60 x 85 mm großen Teebeutel eingewogen, der anschließend verschweißt wird. Der Teebeutel wird für 30 Minuten in einen Überschuß von 0,9 gew.-%igen Kochsalzlösung gegeben (mindestens 0,83 1 Kochsalz-Lösung/1 g Polymerpulver). Anschließend wird der Teebeutel 3 Minuten lang bei 250 g zentrifugiert. Die Bestimmung der Flüssigkeitsmenge geschieht durch Auswägen des zentrifugierten Teebeutels.

Absorption unter Druck (AUL - Absorbency Under Load) (0.7 psi)

Die Meßzelle zur Bestimmung der AUL 0.7 psi stellt ein Plexiglas-Zylinder mit einem Innendurchmesser von 60 mm und einer Höhe von 50 mm dar, der an der Unterseite einen angeklebten Edelstahl-Siebboden mit einer Maschenweite von 36 μm besitzt. Zu der Meßzelle gehört weiterhin eine Plastikplatte mit einem Durchmesser von 59 mm und ein Gewicht, welches zusammen mit der Plastikplatte in die Meßzelle hineingestellt werden kann. Das Gewicht der Plastikplatte und das Gewicht betragen zusammen 1345 g. Zur Durchführung der Bestimmung der AUL 0.7 psi wird das Gewicht des leeren Plexiglas-Zylinders und der Plastikplatte ermittelt und als $W_o$ notiert. Dann werden 0,900 $\pm$ 0,005 g Hydro-formendes Polymer (Korngrößenverteilung 150-800 μm) in den Plexiglas-Zylinder eingewogen und möglichst gleichmäßig auf dem Edelstahl-Siebboden verteilt. Anschließend wird die Plastikplatte vorsichtig in den Plastik-Zylinder hineingelegt und die gesamte Einheit gewogen; das Gewicht wird als $W_a$ notiert. Nun wird das Gewicht auf die Plastikplatte in dem Plexiglas-Zylinder gestellt. In der Mitte der Petrischale mit einem Durchmesser von 200 mm und einer Höhe von 30 mm wird eine keramische Filterplatte mit einem Durchmesser von 120 mm und einer Porosität 0 gelegt und soviel 0,9 gew.-%ige Natriumchloridlösung eingefüllt, daß die Flüssigkeitsoberfläche mit der Filterplattenoberfläche abschließt, ohne daß die Oberfläche der Filterplatte benetzt wird. Anschließend wird ein rundes Filterpapier mit einem Durchmesser von 90 mm und einer Porengröße < 20 μm (S&S 589 Schwarzband von Schleicher & Schüll) auf die keramische Platte gelegt. Der Hydrogel-formendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als $W_b$ notiert.

Die Absorption unter Druck (AUL) wird wie folgt berechnet:

$$\texttt{AUL 0.7 psi [g/g] = [W}_b\texttt{-W}_a\texttt{]/[W}_a\texttt{-W}_0\texttt{]}$$

Saline Flow Conductivity (SFC)

Die Testmethode zur Bestimmung der SFC ist in US-A-5,599,335.

Messung des pH-Wertes der Hydrogel-formenden Polymere

100 ml 0,9 gew.-%ige NaCl-Lösung werden in einem 150 ml-Becherglas mit Hilfe eines Magnetrührers mit moderater Geschwindigkeit gerührt, so daß durch die Rührung keine Luft in die Lösung eingezogen wird. Zu dieser Lösung werden 0,5 $\pm$ 0,001 g Hydrogel-formendes Polymer gegeben und 10 Minuten lang gerührt. Nach 10 Minuten wird der pH der Lösung mit Hilfe einer pH-Glaselektrode gemessen, wobei der Wert erst dann abgelesen wird, wenn er stabil ist, frühestens jedoch nach 1 Minute.

Ammoniakbestimmung zur Geruchskontrolle

Die Ermittlung des Ammoniak-Stickstoffgehaltes erfolgt kolorimetrisch nach der Nessler-Methode. Die Durchführung der Messung erfolgte am DR/2000 Spektrometer der Hach Company, Loveland, CO 80539, USA.

5 g der verschiedenen Superabsorber-Proben wurden mit 600 ml einer 0,9 %igen NaCl und 1,8 %igen Harnstofflösung für 20 min getränkt. Die Lösungen wurden abfiltriert und 25 ml der Lösung wurde mit 10 μl Urease Lösung versetzt. Nach 2 Minuten wurde Stickstoff aus Ammoniak nach der Nessler Methode bestimmt.

Aus Harnstoff wird durch Urease Ammoniak abgespalten; es entwickelt sich eine gelbe Farbe proportional zur Ammoniakkonzentration. Da die Urease-Aktivität stark pH-Wert-abhängig ist, und die größte Aktivität bei pH = 6,1 erreicht ist, ist bei niedrigerem pH eine geringere Ammoniak-Entwicklung zu erwarten. Bei saurem SAP sollte also eine geringe Ammoniakkonzentration zu messen sein.

Stärke der Fixierung von SAP an die Fasermatrix

Zur Bestimmung der Fixierung des superabsorbierenden Polymermatierals in der absorbierenden Komposition wurden im Labor Pads aus Cellulosefasermaterial und hochquellfähigem Polymermaterial (SAP) hergestellt. Dabei gelangten sowohl saures als auch teilneutralisiertes superabsorbierendes Polymermaterial zum Einsatz. Der mittlere Teilchendurchmesser der jeweils eingesetzten SAP betrug 100 bis 800 μm.

Um die Stärke der Fixierung des Polymermaterials an die Fasermatrix zu bestimmen, wurde der Pad in eine Siebmaschine der Firma Retsch (Siebboden: 2 mm Maschenweite) überführt und bei einer Schüttelintensität von 90 % über eine Zeitdauer von 5 Minuten gerüttelt. Im Anschluß daran wurde der abgesiebte SAP gewogen. Als Maß für die Stärke der Fixierung diente der prozentuale Anteil an partikulärem SAP im Pad nach dem Schütteln.

Herstellung von teilneutralen, teilchenförmigen SAP Polymer

Polymer I

In einen mittels Vakuumpumpe auf 980 mbar absolut evakuierten Laborkneter mit einem Arbeitsvolumen von 2 1 (WERNER + PFLEIDERER) wurde eine zuvor separat hergestellte, auf ca. 25°C abgekühlte und durch Einleiten von Stickstoff inertisierte Monomerlösung eingesaugt. Die Monomerlösung setzte sich wie folgt zusammen: 825,5 g Wasser, 431 g Acrylsäure, 120,68 g NaOH 50 %ig, 0,86 g Polyethylenglykol-400-diacrylat (SARTOMER® 344 der Firma CRAY VALLEY). Zur besseren Inertisierung wurde der Kneter evakuiert und anschließend mit Stickstoff belüftet. Dieser Vorgang wurde 3 x wiederholt. Anschließend wurde eine Lösung aus 1,2 g Natriumpersulfat, gelöst in 6,8 g VE-Wasser und nach weiteren 30 Sekunden eine weitere Lösung, bestehend aus 0,024 g Ascorbinsäure, gelöst in 4,8 g Wasser eingesaugt. Es wurde mit Stickstoff gespült. Ein auf 75°C vorgeheizter Mantelheizkreislauf (Bypass) wurde auf den Knetermantel umgestellt und die Rührerdrehzahl auf 96 UpM erhöht. Nach einsetzender Polymerisation und Erreichen von $T_{max}$ wurde der Mantelheizkreislauf wieder auf Bypass umgestellt und 15 Minuten ohne Heizung/Kühlung nachpolymerisiert, anschließend gekühlt und das Produkt ausgetragen. Die entstandenen Gelpartikel trocknete man auf Blechen mit Drahtnetzboden bei 160°C im Umlufttrockenschrank. Danach wurden sie gemahlen und gesiebt.

1200 g des so erhaltenen Produktes der Kornverteilung 105-850 $\mu$m wurden in einem Pulvermischaggregat (Loedige-Mischer) mit einer homogenen Lösung aus 17,58 g Wasser, 9,96 g Propandiol-1,2, 1,2 g Ethylenglykoldiglycidylether und 3,36 g einer 26,8 %igen wässrigen Aluminiumsulfat-Lösung besprüht und in einen vorgewärmten 2. Lödig-Mischer umgefüllt. Unter konstant gehaltenen Bedingungen bei 150°C Manteltemperatur und einer Umdrehungszahl von 60 UpM erfolgte die Temperung über einen Zeitraum von 70 Minuten. Der Mischer wurde entleert, das Produkt auf Raumtemperatur gekühlt und bei 105/850 $\mu$m abgesiebt, um eventuell entstandene Agglomerate oder entstandene Feinanteile zu entfernen. Die Performancedaten des SAP sind der Tabelle 1 zu entnehmen.

Herstellung teilneutralen, teilchenförmigen SAP

Polymer II

Beim teilneutralen hochquellfähigen Polymer handelt es sich um handelsübliches TYLOSE VS 3790 (Lot./Avil 903642), ein Superabsorber der CASSELLA AG - Frankfurt am Main, mit einem pH von 5-5,5 (hergestellt analog Beispiel 7, EP-B-0316792). Das Polymer wurde im 20 g Maßstab in einem WARNING-Blender (modifizierter Aufsatz für Küchenmixer) mittels einer 2 ml-Spritze mit einer Oberflächennachvernetzungslösung aus 2,3 % Wasser/1,2 % Propandiol-1,2/0,2 % Ethylenglykoldiglycidylether (jeweils bezogen auf Polymer) versetzt und 1 Stunde bei 140°C in einem Umlufttrockenschrank getempert. Die Performancedaten sind in Tabelle 1 angegeben.

Tabelle 1

|  | pH | SFC x $10^{-7}$ cm$^3$s/g | CRC g/g | AUL 0.7 psi g/g | $N_2$ aus $NH_3$ (Nessler) mg/l | Neutralisationsgrad [mol-%] |
|---|---|---|---|---|---|---|
| Polymer I | 4,3 | 13,8 | 20,7 | 18,1 | 1,8 | 25 |
| Polymer II | 5,4 | 17 | 32,9 | 23,0 | 5,6 | 45 |

Herstellung von Pads

Die verwendete Apparatur bestand aus einem rechteckigen Plexiglasgefäß der Abmessungen 10 x 15 cm, das unten durch ein Edelstahlsieb der Maschenweite 1 mm abgeschlossen wurde.
Unterhalb des Edelstahlsiebes wurde zur Erzeugung eines Unterdrucks ein handelsüblicher Staubsauger angeschlossen.

Herstellung von Pads mit homogenen Lagen

Zur Herstellung inhomogener Lagen wurden in die oben beschriebene Apparatur bei Unterdruck 4 g Cellulosefasermaterial, das mit 6 g hochquellfähigem SAP vermischt war, auf einmal zugegeben. Der Pad wies eine gleichmäßige Dicke auf.

Die in den Beispielen angegebenen Teile sind Gewichtsteile, die Angaben in % sind Gewichtsprozent, sofern aus dem Zusammenhang nichts anderes hervorgeht.

Beispiel 1

Nach der oben beschriebenen Methode wurde ein Pad mit folgender Schichtung hergestellt: 2 g Cellulosefasermaterial und 6 g Polymer I. Anschließend wurde diese Schichtung mit Triethanolamin besprüht, bis der Neutralisationsgrad der Carboxylgruppen im Polymer I 80 Mol-% betrug (Pad 1). In einem zweiten Schritt wurden 2 g Cellulosefasermaterial in die Apparatur eingefüllt und ebenfalls mit Triethanolamin besprüht (Pad 2). Danach legt man Pad 2 mit der besprühten Seite auf Pad 1 und presst diesen zusammengelegten Pad zweimal 15 s bei 200 bar.

Beispiel 2

Beispiel 1 wurde mit der einzigen Ausnahme wiederholt, daß man dieselbe Menge an Polymer II einsetzte.

Beispiel 3

Man stellte in der oben beschriebenen Apparatur einen Pad mit inhomogener Schichtung her, indem man eine Mischung aus 2 g Cellulosefasern und 6 g Polymer I in die Apparatur einfüllte und anschließend 2 g Cellulosefasern als Schicht aufbrachte. Durch Besprühen der Schichtung mit Triethanolamin wurde das Polymer I bis zu einem Neutralisationsgrad von 80 Mol-% neutralisiert.

Beispiel 4

Beispiel 1 wurde mit der einzigen Ausnahme wiederholt, daß man den Neutralisationsgrad der Carboxylgruppen im Polymer I auf 60 Mol-% einstellte.

Beispiel 5

Nach der oben beschriebenen Methode wurde ein Pad mit homogener Schichtung hergestellt, indem man zunächst 1 g Cellulosefasermaterial, danach 8 g Polymer I und anschließend 1 g Cellulosefasermaterial in die Apparatur einfüllte. Die so erhaltene Schichtung wurde mit einer solchen Menge von Triethanolamin besprüht, bis der Neutralisationsgrad der Carboxylgruppen im Polymer I 80 Mol-% betrug.

Vergleichsbeispiel 1

Beispiel 1 wurde mit der einzigen Ausnahme wiederholt, daß man die Carboxylgruppen des Polymer I durch Besprühen der Schichtung mit 50 %-iger wäßriger Natronlauge bis zu einem Neutralisationsgrad von 80 Mol-% neutralisierte.

Vergleichsbeispiel 2

Beispiel 2 wurde mit der einzigen Ausnahme wiederholt, daß man anstelle einer wäßrigen Lösung von Triethanolamin eine 50 %-ige wäßrige Natronlauge einsetzte und die Carboxylgruppen des Polymer II bis zu einem Neutralisationsgrad von 80 % neutralisierte.

Die nach den Beispielen und den Vergleichsbeispielen hergestellten Komposits wurden bei einer Temperatur von 80°C getrocknet und anschließend auf die Stärke der Fixierung von SAP-Teilchen nach der oben beschriebenen Methode untersucht. Die Ergebnisse sind in Tabelle 2 angegeben.

Tabelle 2

| Beispiel | Schichtung im Pad | Polymer-Anteil im Pad | Gesamtneutralisationsgrad | Siebtest Polymer-Anteil abgesiebt |
|---|---|---|---|---|
| 1 | homogen | Polymer I, 60 % | 80 Mol-% | 6 % |
| 2 | homogen | Polymer II, 60 % | 80 Mol-% | 11 % |
| 3 | inhomogen | Polymer I, 60 % | 80 Mol-% | 5 % |

(fortgesetzt)

| Beispiel | Schichtung im Pad | Polymer-Anteil im Pad | Gesamtneutralisationsgrad | Siebtest Polymer-Anteil abgesiebt |
|---|---|---|---|---|
| 4 | homogen | Polymer I, 60 % | 60 Mol-% | 9 % |
| 5 | homogen | Polymer I, 80 % | 80 Mol-% | 8 % |
| Vergleichsbeispiel 1 | homogen | Polymer I, 60 % | 80 Mol-% | 35 % |
| Vergleichsbeispiel 2 | homogen | Polymer II, 60 % | 80 Mol-% | 42 % |

**Patentansprüche**

1. Verfahren zum Binden von teilchenförmigen, wasserabsorbierenden, Säuregruppen enthaltenden Polymeren an ein Trägermaterial mit Hilfe von Aminogruppen aufweisenden Verbindungen, **dadurch gekennzeichnet, daß** man das Trägermaterial mit teilchenförmigen, wasserabsorbierenden, Säuregruppen enthaltenden Polymeren in Kontakt bringt, deren Säuregruppen zu 0 bis höchstens 55 Mol-% mit Alkalimetall- und/oder Ammoniumbasen neutralisiert sind, und dann den Neutralisationsgrad dieser Polymeren durch Behandlung mit Aminogruppen enthaltenden Verbindungen auf mindestens 60 Mol-% erhöht.

2. Verfahren nach Anspruch 1, **dadurch gekennzeichnet, daß** man teilchenförmige, wasserabsorbierende Polymere einsetzt, deren Säuregruppen zu weniger als 50 Mol-% neutralisiert sind.

3. Verfahren nach Anspruch 1 oder 2, **dadurch gekennzeichnet, daß** man teilchenförmige, wasserabsorbierende Polymere einsetzt, deren Säuregruppen zu 0 bis 45 Mol-% neutralisiert sind.

4. Verfahren nach einem der Ansprüche 1 bis 3, **dadurch gekennzeichnet, daß** man teilchenförmige, wasserabsorbierende Polymere einsetzt, deren Säuregruppen zu 15 bis 40 Mol-% - neutralisiert sind.

5. Verfahren nach einem der Ansprüche 1 bis 3, **dadurch gekennzeichnet, daß** man teilchenförmige, wasserabsorbierende Polymere einsetzt, bei denen sämtliche Säuregruppen in Form von freien Carboxylgruppen vorliegen.

6. Verfahren nach einem der Ansprüche 1 bis 5, **dadurch gekennzeichnet, daß** man als Aminogruppen enthaltende Verbindungen Alkanolamine einsetzt.

7. Verfahren nach einem der Ansprüche 1 bis 6, **dadurch gekennzeichnet, daß** man als Aminogruppen enthaltende Verbindung Triethanolamin einsetzt.

8. Verfahren nach einem der Ansprüche 1 bis 6, **dadurch gekennzeichnet, daß** man als Aminogruppen enthaltende Verbindungen Ethylenimin- und/oder Vinylamineinheiten enthaltende Verbindungen einsetzt.

9. Verfahren nach Anspruch 8, **dadurch gekennzeichnet, daß** man als Aminogruppen enthaltende Verbindungen Polymere mit einer Molmasse Mw von mindestens 500 einsetzt.

10. Verfahren nach Anspruch 8, **dadurch gekennzeichnet, daß** man als Aminogruppen enthaltende Verbindungen Polyethylenimine mit Molmassen Mw von 1000 bis 2 Millionen, vorzugsweise 1500 bis 500.000 einsetzt.

11. Verfahren nach Anspruch 8, **dadurch gekennzeichnet, daß** man als Aminogruppen enthaltende Verbindungen Vinylamineinheiten enthaltende Polymerisate einsetzt, die durch partielle oder vollständige Hydrolyse von Homo- oder Copolymerisaten von N-Vinylformamid erhältlich sind.

12. Verfahren nach Anspruch 8, **dadurch gekennzeichnet, daß** man als Aminogruppen enthaltende Verbindungen Polyvinylamin einsetzt.

13. Verfahren nach einem der Ansprüche 1 bis 12, **dadurch gekennzeichnet, daß** der Neutralisationsgrad der Säuregruppen enthaltenden Polymeren nach der Behandlung mit Aminogruppen enthaltenden Verbindungen 60 bis 95 Mol-% beträgt.

14. Verfahren nach einem der Ansprüche 1 bis 12, **dadurch gekennzeichnet, daß** der Neutralisationsgrad der Säuregruppen enthaltenden Polymeren nach der Behandlung mit Aminogruppen enthaltenden Verbindungen 65 bis 85 Mol-% beträgt.

15. Verfahren nach einem der Ansprüche 1 bis 7, **dadurch gekennzeichnet, daß** man teilchenförmige, vernetzte Polyacrylsäuren mit einem Neutralisationsgrad von 0 bis weniger als 50 Mol-% mit Cellulosefasern mischt und die Mischung mit einer solchen Menge an Triethanolamin behandelt, daß der Neutralisationsgrad der Säuregruppen enthaltenden Polymeren auf 65 bis 85 Mol-% erhöht wird.

16. Verfahren nach einem der Ansprüche 1 bis 15, **dadurch gekennzeichnet, daß** die Trägermaterialien ausgewählt sind aus der Gruppe bestehend aus natürlichen Fasern, synthetischen Fasern, deren Mischungen, Vliesen aus den genannten Fasern und Folien.

17. Trägermaterial mit teilchenförmigen, wasserabsorbierenden, Säuregruppen enthaltenden Polymeren erhältlich nach einem Verfahren einer der Ansprüche 1 bis 16.

18. Hygieneartikel enthaltend ein Trägermaterial gemäß Anspruch 17.

19. Verwendung von Aminogruppen aufweisenden Verbindungen aus der Gruppe der Alkanolamine, Ethylenimin- und/ oder Vinylamineinheiten enthaltenden Verbindungen zur Neutralisation und zum Binden von teilchenförmigen, wasserabsorbierenden, Säuregruppen enthaltenden Polymeren, die zu 0 bis höchstens 55 Mol-% mit Alkalimetall- und/ oder Ammoniumbasen neutralisiert sind, an ein Trägermaterial.

**Claims**

1. A process for binding particulate water-absorbing acid-functional polymers to a carrier material by means of compounds comprising amino groups, which comprises contacting the carrier material with particulate water-absorbing acid-functional polymers whose acid groups have been 0 to not more than 55 mol% neutralized with alkali metal and/or ammonium bases and then raising the degree of neutralization of these polymers to not less than 60 mol% by treatment with amino-containing compounds.

2. The process according to claim 1 that utilizes particulate water-absorbing polymers whose acid groups are less than 50 mol% neutralized.

3. The process according to claim 1 or 2 that utilizes particulate water-absorbing polymers whose acid groups are 0 to 45 mol% neutralized.

4. The process according to any of claims 1 to 3 that utilizes particulate water-absorbing polymers whose acid groups are 15 to 40 mol% neutralized.

5. The process according to any of claims 1 to 3 that utilizes particulate water-absorbing polymers where all the acid groups are present in the form of free carboxyl groups.

6. The process according to any of claims 1 to 5 that utilizes alkanolamines as amino-containing compounds.

7. The process according to any of claims 1 to 6 that utilizes triethanolamine as amino-containing compounds.

8. The process according to any of claims 1 to 6 that utilizes compounds comprising ethyleneimine and/or vinylamine units as amino-containing compounds.

9. The process according to claim 8 that utilizes polymers having a molar mass $M_w$ of at least 500 as amino-containing compounds.

10. The process according to claim 8 that utilizes polyethyleneimines having molar masses $M_w$ of 1000 to 2 million and preferably 1500 to 500 000 as amino-containing compounds.

11. The process according to claim 8 wherein the amino-containing compounds used are addition polymers comprising vinylamine units and obtainable by partial or complete hydrolysis of homo- or copolymers of N-vinylformamide.

12. The process according to claim 8 that utilizes polyvinylamine as amino-containing compounds.

13. The process according to any of claims 1 to 12 wherein the degree of neutralization of the acid-functional polymers after the treatment with amino-containing compounds is in the range from 60 to 95 mol%.

14. The process according to any of claims 1 to 12 wherein the degree of neutralization of the acid-functional polymers after the treatment with amino-containing compounds is in the range from 65 to 85 mol%.

15. The process according to any one of claims 1 to 7 wherein particulate crosslinked polyacrylic acids having a degree of neutralization in the range from 0 to less than 50 mol% are mixed with cellulose fibers and the mixture is treated with such an amount of triethanolamine that the degree of neutralization of the acid-functional polymers is raised to 65-85 mol%.

16. The process according to any one of claims 1 to 15 wherein the carrier materials are selected from the group consisting of natural fibers, synthetic fibers, their blends, webs composed of said fibers and films/sheets.

17. A carrier material supporting particulate water-absorbing acid-functional polymers obtainable by a process of any of claims 1 to 16.

18. A hygiene article comprising a carrier material according to claim 17.

19. The use of compounds comprising amino groups and selected from the group of the alkanolamines and compounds comprising ethyleneimine and/or vinylamine units to neutralize and bind particulate water-absorbing acid-functional polymers neutralized 0 to not more than 55 mol% with alkali metal and/or ammonium bases to a carrier material.

**Revendications**

1. Procédé de fixation de polymères particulaires, absorbant l'eau et contenant des groupes acides sur une matière de support à l'aide de composés présentant des groupes amino, **caractérisé en ce qu'**on met en contact la matière de support avec des polymères particulaires, absorbant l'eau et contenant des groupes acides, dont les groupes acides sont neutralisés à 0 jusqu'à au maximum 55 % molaires par des bases de métal alcalin et/ou d'ammonium, et ensuite on augmente le degré de neutralisation de ces polymères à au moins 60 % molaires par traitement avec des composés contenant des groupes amino.

2. Procédé suivant la revendication 1, **caractérisé en ce qu'**on met en oeuvre des polymères particulaires, absorbant l'eau, dont les groupes acides sont neutralisés à moins de 50 % molaires.

3. Procédé suivant la revendication 1 ou 2, **caractérisé en ce qu'**on met en oeuvre des polymères particulaires, absorbant l'eau, dont les groupes acides sont neutralisés à 0 jusqu'à 45 % molaires.

4. Procédé suivant l'une des revendications 1 à 3, **caractérisé en ce qu'**on met en oeuvre des polymères particulaires, absorbant l'eau, dont les groupes acides sont neutralisés à 15 jusqu'à 40 % molaires.

5. Procédé suivant l'une des revendications 1 à 3, **caractérisé en ce qu'**on met en oeuvre des polymères particulaires, absorbant l'eau, dans lesquels l'ensemble des groupes acides se présentent sous la forme de groupes carboxyle libres.

6. Procédé suivant l'une des revendications 1 à 5, **caractérisé en ce que**, comme composés comprenant des groupes amino, on met en oeuvre des alcanolamines.

7. Procédé suivant l'une des revendications 1 à 6, **caractérisé en ce que**, comme composé contenant des groupes amino, on met en oeuvre de la triéthanolamine.

**8.** Procédé suivant l'une des revendications 1 à 6, **caractérisé en ce que**, comme composés contenant des groupes amino, on met en oeuvre des composés contenant des unités éthylèneimine et/ou vinylamine.

**9.** Procédé suivant la revendication 8, **caractérisé en ce que**, comme composés contenant des groupes amino, on met en oeuvre des polymères présentant une masse molaire Mw d'au moins 500.

**10.** Procédé suivant la revendication 8, **caractérisé en ce que**, comme composés contenant des groupes amino, on met en oeuvre des polyéthylèneimines présentant des masses molaires Mw de 1000 à 2 millions, de préférence de 1500 à 500 000.

**11.** Procédé suivant la revendication 8, **caractérisé en ce que**, comme composés contenant des groupes amino, on met en oeuvre des polymères contenant des unités vinylamine qui peuvent être obtenus par hydrolyse partielle ou totale d'homopolymères ou de copolymères de N-vinylformamide.

**12.** Procédé suivant la revendication 8, **caractérisé en ce que**, comme composés contenant des groupes amino, on met en oeuvre de la polyvinylamine.

**13.** Procédé suivant l'une des revendications 1 à 12, **caractérisé en ce que** le degré de neutralisation des polymères contenant des groupes acides est de 60 à 95 % molaires après le traitement avec des composés contenant des groupes amino.

**14.** Procédé suivant l'une des revendications 1 à 12, **caractérisé en ce que** le degré de neutralisation des polymères contenant des groupes acides est de 65 à 85 % molaires après le traitement avec des composés contenant des groupes amino.

**15.** Procédé suivant l'une des revendications 1 à 7, **caractérisé en ce qu'**on mélange des acides polyacryliques particulaires, réticulés, présentant un degré de neutralisation de 0 à moins de 50 % molaires avec des fibres de cellulose et on traite le mélange avec une quantité de triéthanolamine telle que le degré de neutralisation des polymères contenant des groupes acides est augmenté à 65 jusqu'à 85 % molaires.

**16.** Procédé suivant l'une des revendications 1 à 15, **caractérisé en ce que** les matières de support sont choisies parmi le groupe constitué de fibres naturelles, de fibres synthétiques, de leurs mélanges, de non-tissés à base des fibres citées et de feuilles.

**17.** Matière de support comportant des polymères particulaires, absorbant l'eau et contenant des groupes acides, que l'on peut obtenir selon un procédé suivant l'une des revendications 1 à 16.

**18.** Article d'hygiène contenant une matière de support suivant la revendication 17.

**19.** Utilisation de composés présentant des groupes amino du groupe des alcanolamines et des composés contenant des unités éthylèneimine et/ou vinylamine, pour la neutralisation et la fixation de polymères particulaires, absorbant l'eau et contenant des groupes acides, qui sont neutralisés à 0 jusqu'au maximum 55 % molaires par des bases de métal alcalin et/ou d'ammonium, sur une matière de support.